# EUROPEAN PATENT APPLICATION

(11) **EP 4 778 486 A1**
(43) Date of publication of application: **22.07.2026**
(21) Application number: 24865720.7
(22) Date of filing: 26.08.2024
(51) Int. Cl.: A61B 34/10, A61C 1/08, A61C 8/00

(54) **METHOD AND APPARATUS FOR DESIGNING LATERAL APPROACH SURGICAL GUIDE**

(30) Priority: 14.09.2023 KR 20230122781
(71) Applicant: Osstem Implant Co., Ltd., Gangseo-gu Seoul 07789 (KR)
(72) Inventor: KIM, Jong Moon, Gunpo-si Gyeonggi-do 15827 (KR)
(74) Representative: Klunker IP Patentanwälte PartG mbB
(86) International application number: PCT/KR2024/012690
(87) International publication number: WO 2025/058279

(57) **Abstract**

Disclosed are a method and an apparatus for designing a lateral approach surgical guide. An embodiment provides a method and an apparatus for designing a lateral approach surgical guide, wherein in a case where a maxillary sinus lift procedure is required using a lateral approach method, a maxillary sinus from dental imaging data is segmented, a bone window is formed on the basis of the segmented maxillary sinus, and then the shape of a lateral approach surgical guide is formed according to the thickness of a residual bone in a bone window formation area and a stopper of a cutting tool. Accordingly, the guide for the lateral approach method can be quickly and accurately generated.

## Description

### [Technical Field]

The present invention relates to dental imaging data processing technology, and more particularly, to techniques for designing a lateral approach surgical guide and for a user interface.

### [Background Art]

Implant surgery for placing artificial teeth is spreading rapidly and is becoming a common procedure in dentistry. However, a significant number of patients have oral structures that make implant surgery difficult, leading to challenges in implant treatment. In particular, implant placement is not easy when there is insufficient residual bone in the posterior maxilla where the maxillary sinus is located. To address this, a maxillary sinus membrane lift procedure may be used. The maxillary sinus membrane lift procedure is a method of elevating a maxillary sinus membrane to create a space, performing bone grafting in the secured space, and placing an implant therein.

One type of the maxillary sinus membrane lift procedures includes a vertical approach. The vertical approach is a method used when residual bone is secured to some extent at the implant treatment site. According to the vertical approach, an operator drills a hole in the maxilla so that the maxillary sinus membrane is not damaged, and then gradually inserts bone graft through the hole. Alternatively, the maxillary sinus membrane is lifted using hydraulic pressure and an implant is placed. However, in the vertical approach, it is difficult for the operator to directly view the maxillary sinus membrane during the procedure. Therefore, the operator must proceed very carefully while checking the procedure via X-ray, and thus there is a drawback in that the procedure time is long. In addition, when perforation of the maxillary sinus occurs during drilling, there is the inconvenience that the procedure has to be switched to a lateral approach.

The lateral approach, which is another type of maxillary sinus membrane lift procedure, is a method used when residual bone at the implant treatment site is very insufficient. According to the lateral approach, the operator forms a window in the lateral side of the maxillary sinus, elevates the maxillary sinus membrane, and then performs bone grafting through the window. Since the lateral approach allows the operator to lift the maxillary sinus membrane while directly viewing it during the procedure, the occurrence of damage to the maxillary sinus membrane can be reduced. Even if damage occurs, post-treatment is possible. In addition, a large amount of bone graft material can be rapidly introduced at one time, so that the processing time can be reduced.

### [Disclosure]

### [Technical problem]

According to an embodiment, a method and an apparatus for designing a lateral approach surgical guide are proposed which make it possible to generate a guide for a lateral approach rapidly and accurately while minimizing user operations.

### [Technical Solution]

A method for designing a lateral approach surgical guide according to an embodiment includes: acquiring dental imaging data of a patient; segmenting, from the acquired dental imaging data, a tooth region including a missing tooth area, and anatomical structures including a maxillary sinus and a maxilla; calculating a thickness of a residual bone on the basis of a distance, measured with respect to a central axis of the missing tooth area, between the maxillary sinus and the maxilla among the segmented anatomical structures; forming a bone window on the basis of the segmented maxillary sinus when the calculated thickness of the residual bone does not exceed a predetermined distance; and generating a lateral approach surgical guide including a guide rail formed at a predetermined distance from a periphery of the formed bone window so as to be larger than the bone window.

The method for designing a lateral approach surgical guide may further include: receiving an implant placement site and a treatment plan; and providing a lateral approach surgical mode when the implant placement site is in a posterior maxilla and the treatment plan is for a maxillary sinus lift procedure.

The segmenting of the anatomical structures may include: determining a size of the missing tooth area from the dental imaging data; displaying the missing tooth area having the determined size as a hexahedron in 3D and as a rectangle in 2D; and arranging the missing tooth area on an arch line.

In the determining of the size of the missing tooth area, when there is no tooth at a most posterior position, the size of the missing tooth area may be determined using an average tooth size from the dental imaging data, and when a contralateral tooth exists on the opposite side of the missing tooth area, the size of the missing tooth area may be determined to be the same as a size of the contralateral tooth.

In the determining of the size of the missing tooth area, where teeth are present on both sides of the missing tooth area, a vacant region between adjacent teeth may be determined as the size of the missing tooth area.

In the determining of the size of the missing tooth area, when there are a plurality of missing teeth, the size of the missing tooth area may be determined by substituting a space between adjacent teeth into an average tooth size.

The forming of the bone window may include: generating an inferior edge of the bone window at a position spaced superiorly by a first distance from an inferior border of the maxillary sinus; generating an anterior edge of the bone window at a position spaced posteriorly by a second distance from an anterior border of the maxillary sinus; generating a superior edge of the bone window at a position spaced superiorly by a third distance from the inferior edge of the bone window; generating a posterior edge of the bone window at a position spaced posteriorly by a fourth distance from the anterior edge of the bone window; and connecting reference points including the generated inferior edge, anterior edge, superior edge, and posterior edge of the bone window to form a quadrilateral, and generating, within the quadrilateral, an ellipse passing through each of the reference points to form the bone window.

In the forming of the bone window, when a tooth is present at a most posterior position, the posterior edge of the bone window may be generated at a predetermined distance anterior to a region of the most posterior tooth.

The forming of the bone window may include, when a septum is present in the dental imaging data, generating a posterior edge of the bone window at a predetermined distance anterior to the septum while regarding the septum as a posterior border of a bone; generating an anterior edge of the bone window at a predetermined distance posterior to the septum while regarding the septum as an anterior border of a bone; additionally generating a superior edge and an inferior edge of the bone window; and connecting reference points including the generated posterior edge, anterior edge, superior edge, and inferior edge of the bone window to form the bone window.

In the generating of the lateral approach surgical guide, a shape of the guide rail may be determined using the thickness of the residual bone and a length of a cutting tool.

The generating of the lateral approach surgical guide may include: when the thickness of the residual bone is greater than the length of the cutting tool, generating the guide rail in a shape having a step on an inner side; and when the thickness of the residual bone is less than the length of the cutting tool, generating the guide rail in a shape protruding outward.

In the generating of the lateral approach surgical guide, the guide rail may be formed at a predetermined distance from the periphery of the bone window so as to be greater than the bone window such that a stopper of a cutting tool for lateral approach is allowed to be positioned thereon.

The generating of the lateral approach surgical guide may include: generating model data in which a maxillary sinus area, the missing tooth area, and a bone serving as a reference for an arch line are exposed; and generating the lateral approach surgical guide from the model data.

In the generating of the lateral approach surgical guide from the model data, a bone-level lateral approach surgical guide may be generated for a bone window formation area, and a gingival-level lateral approach surgical guide may be generated for a remaining area including a drilling hole area for placing an implant.

In the generating of the lateral approach surgical guide, a thickness of the guide rail for forming the bone window in the lateral approach surgical guide may be determined by adjusting a step of the guide rail such that a stopper of the cutting tool can contact the guide rail at predefined discrete steps in accordance with a length of the stopper of the cutting tool and the thickness of the residual bone in the bone window formation area.

The method for designing a lateral approach surgical guide may further include: recommending generation of the lateral approach surgical guide when the calculated thickness of the residual bone does not exceed a predetermined distance; and forming the bone window on the basis of the segmented maxillary sinus upon receiving, from a user, a user operation signal selecting generation of the lateral approach surgical guide.

An apparatus for designing a lateral approach surgical guide according to another embodiment includes: a data acquisition unit configured to acquire dental imaging data for lateral approach surgery; a control unit configured to segment, from the acquired dental imaging data, a tooth region including a missing tooth area, and anatomical structures including a maxillary sinus and a maxilla, calculate a thickness of a residual bone on the basis of a distance, measured with respect to a central axis of the missing tooth area, between the maxillary sinus and the maxilla among the segmented anatomical structures, form a bone window on the basis of the segmented maxillary sinus when the calculated thickness of the residual bone falls within a predetermined distance, and generate a lateral approach surgical guide including a guide rail formed at a predetermined distance from a periphery of the formed bone window so as to be greater than the bone window; and a display unit configured to display data including the lateral approach surgical guide.

### [Effects of the Invention]

According to a method and apparatus for designing a lateral approach surgical guide according to an embodiment, in a case where a maxillary sinus lift procedure is required using a lateral approach method, a maxillary sinus from dental imaging data may be segmented and a bone window may be formed on the basis of the segmented maxillary sinus. Accordingly, the bone window can be generated quickly and accurately, thereby preventing tearing of a maxillary sinus membrane.

In addition, according to the method and apparatus for designing a lateral approach surgical guide, a shape of a lateral approach surgical guide can be formed according to the thickness of a residual bone in a bone window formation area and a stopper of a cutting tool. Accordingly, the guide for the lateral approach method can be generated while minimizing user operations.

Furthermore, the method and apparatus for designing a lateral approach surgical guide can generate the lateral approach surgical guide so as to avoid a position of an artery, thereby ensuring safety.

### [Description of Drawings]

FIG. 1 is a diagram illustrating a configuration of an apparatus for designing a lateral approach surgical guide according to an embodiment of the present invention.
FIG. 2 is a flowchart illustrating a method of designing a lateral approach surgical guide according to an embodiment of the present invention.
FIG. 3 illustrates CT data and scan data according to an embodiment of the present invention.
FIG. 4 illustrates an anatomical structure segmentation screen according to an embodiment of the present invention.
FIG. 5 illustrates a registration screen of CT data and scan data according to an embodiment of the present invention.
FIG. 6 illustrates the size of a missing tooth area according to an embodiment of the present invention.
FIGS. 7 to 10 are views illustrating arch line generation screens according to an embodiment of the present invention.
FIG. 11 illustrates a screen for arranging a missing tooth area on an arch line according to an embodiment of the present invention.
FIG. 12 illustrates a screen for calculating a thickness of the residual bone according to an embodiment of the present invention.
FIGS. 13 to 15 illustrate a screen for generating a bone window according to an embodiment of the present invention.
FIG. 16 illustrates a screen for generating a bone window in a case where a septum is present according to an embodiment of the present invention.
FIG. 17 illustrates a screen for generating a bone window and a guide rail in a case where a septum is present according to an embodiment of the present invention.
FIGS. 18 and 19 illustrate various shapes of a guide rail determined according to the length of a cutting tool according to various embodiments of the present invention.
FIG. 20 illustrates a screen for generating a guide rail in a case where a septum is present according to an embodiment of the present invention.
FIG. 21 illustrates an implant placement screen according to an embodiment of the present invention.
FIG. 22 illustrates a drilling hole formation screen according to an embodiment of the present invention.
FIG. 23 illustrates a screen for generating a gingival-level lateral approach surgical guide according to an embodiment of the present invention.
FIG. 24 illustrates a screen for generating a bone-level lateral approach surgical guide according to an embodiment of the present invention.

### [Mode of the Invention]

The advantages and features of the present invention and the manner of achieving the advantages and features will become apparent with reference to embodiments described in detail below together with the accompanying drawings. However, the present invention may be implemented in many different forms and should not be construed as being limited to the embodiments set forth herein, and the embodiments are provided such that this disclosure will be thorough and complete and will fully convey the scope of the present invention to those skilled in the art, and the present invention is defined only by the scope of the appended claims. Throughout the entire specification, the same or like reference numerals designate the same or like elements.

In describing the example embodiments, a detailed description of related known configurations or functions incorporated herein will be omitted when it is determined that the detailed description thereof may unnecessarily obscure the subject matter of the present invention. The terms which will be described below are terms defined in consideration of the functions in the present invention, and may be different according to users, intentions of the users, or customs. Therefore, the definitions of the terms used herein should follow contexts disclosed herein.

Hereinafter, embodiments of the present invention will be described in detail with reference to the accompanying drawings. However, the example embodiments of the present invention may be modified in various different forms, and the scope of the present invention is not limited to the example embodiments described below. Embodiments of the present invention are provided to aid those skilled in the art in the explanation and the understanding of the present invention.

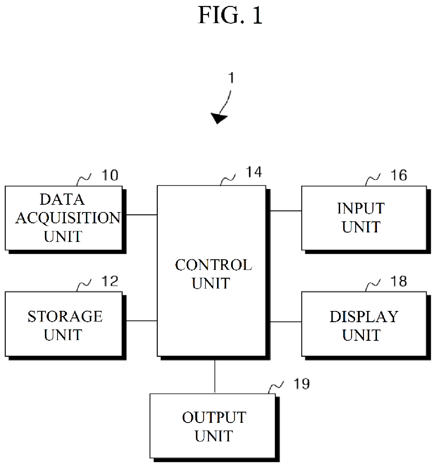

FIG. 1 is a diagram illustrating a configuration of an apparatus for designing a lateral approach surgical guide according to an embodiment of the present invention.

Referring to FIG. 1, the apparatus 1 for designing a lateral approach surgical guide is an electronic device capable of executing a medical image processing program. The electronic device may include a personal computer (PC), a notebook computer, a tablet computer, a smartphone, and the like. Examples of the medical image processing program include a guide design program, a scanning program, a CAD program, and the like. In addition, the program may be applied not only to dental implant surgery but also to general medical image processing applications.

The present invention relates to a program for designing a lateral approach surgical guide to perform a maxillary sinus lift procedure on a patient for whom dental implant placement is difficult due to insufficient residual bone in a posterior maxilla where the maxillary sinus is located. However, the present invention is of course equally applicable to other programs as long as image processing is possible.

In a case where a maxillary sinus lift procedure is required using a lateral approach, the apparatus 1 for designing a lateral approach surgical guide segments anatomical structures including the maxillary sinus from dental imaging data. Then, the apparatus 1 for designing a lateral approach surgical guide forms a bone window on the basis of the segmented maxillary sinus. Subsequently, the apparatus 1 for designing a lateral approach surgical guide generates a shape of a lateral approach surgical guide according to the thickness of a residual bone in a bone window formation area and the length of a cutting tool. The lateral approach method is a technique in which a bone window is formed on a lateral side of the maxillary sinus, the maxillary sinus membrane is elevated, and then bone is grafted. Examples of the cutting tool include drills such as a core drill and a dome drill. A stopper of the cutting tool serves to prevent the cutting tool from advancing further when bone removal by the cutting tool has been performed up to a certain height.

The apparatus 1 for designing a lateral approach surgical guide according to an embodiment includes a data acquisition unit 10, a storage unit 12, a control unit 14, an input unit 16, a display unit 18, and an output unit 19.

The data acquisition unit 10 obtains dental image data. The data acquisition unit 10 may acquire dental image data through a linked medical imaging device (not shown) or a separate database, etc. The dental image data may include CT data, scan data, and the like.

The input unit 16 receives a user operation signal. At this time, the input unit 16 may receive a user operation signal for a user interface displayed on a screen.

The display unit 18 displays the results processed by the control unit 14 on the screen.

The output unit 19 may output a lateral approach surgical guide shape designed by the control unit 14 using a three-dimensional (3D) printer or milling device. Here, the designed lateral approach surgical guide shape may be in a data format compatible with the 3D printer or milling device, and the 3D printer or milling device may output an actual lateral approach surgical guide based on the input lateral approach surgical guide shape.

The storage unit 12 stores various types of data, including information necessary for operating the device 1 for designing a lateral approach surgical guide and information generated during operation. For example, the storage unit 12 may store dental image data for individual patients and provide the data to the control unit 14 upon user request.

The control unit 14 establishes a lateral approach surgical plan from the dental imaging data and generates a lateral approach surgical guide on the dental imaging data according to the established surgical plan. To this end, the control unit 14 segments anatomical structures including the maxillary sinus from the dental imaging data and forms a bone window on the basis of the segmented maxillary sinus. Then, the control unit 14 generates the shape of a guide rail for the lateral approach surgical guide according to the thickness of a residual bone in the bone window formation area and the length of the cutting tool.

The shape of the lateral approach surgical guide generated through the control unit 14 may be output as an actual lateral approach surgical guide in a 3D form through the output unit 19. The shape of the lateral approach surgical guide generated by the control unit 14 may be displayed on the screen of the display unit 18.

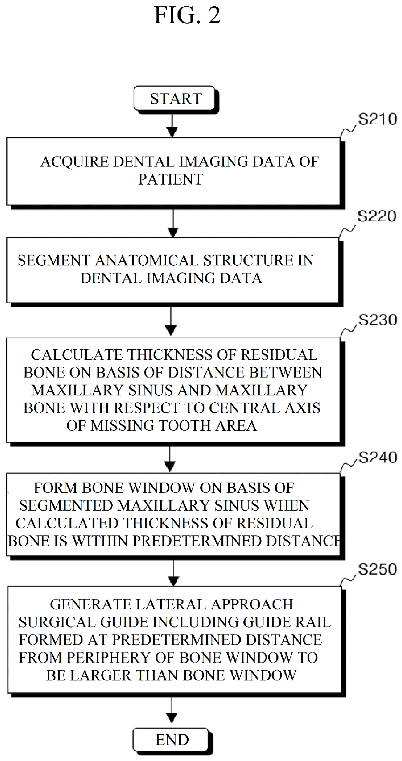

FIG. 2 is a flowchart illustrating a method of designing a lateral approach surgical guide according to an embodiment of the present invention.

Referring to FIGS. 1 and 2, the apparatus 1 for designing a lateral approach surgical guide may acquire an implant placement site and a treatment plan of a patient before acquiring dental imaging data of the patient. At this time, if the implant placement site is in a posterior maxilla and the treatment plan is a maxillary sinus lift procedure, the apparatus 1 for designing a lateral approach surgical guide provides a lateral approach surgical mode to the user.

The posterior maxilla includes teeth #14 (maxillary right first premolar), #15 (maxillary right second premolar), #16 (maxillary right first molar), #17 (maxillary right second molar), #24 (maxillary left first premolar), #25 (maxillary left second premolar), #26 (maxillary left first molar), #27 (maxillary left second molar), and the like.

Subsequently, the apparatus 1 for designing a lateral approach surgical guide acquires dental imaging data of a patient (S210). The dental imaging data may include CT data, scan data, and the like.

Subsequently, the apparatus 1 for designing a lateral approach surgical guide segments anatomical structures from the acquired dental imaging data (S220). The anatomical structures include a tooth region including a missing tooth area, the maxillary sinus, the maxilla, and the like.

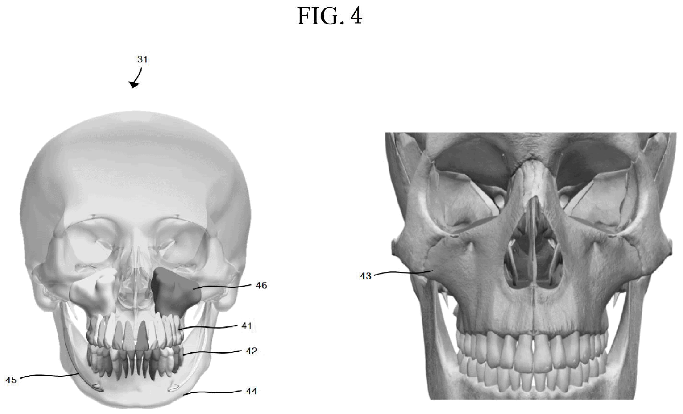

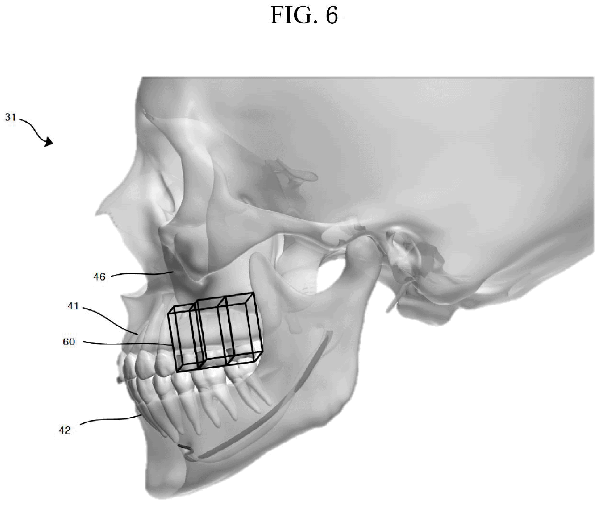

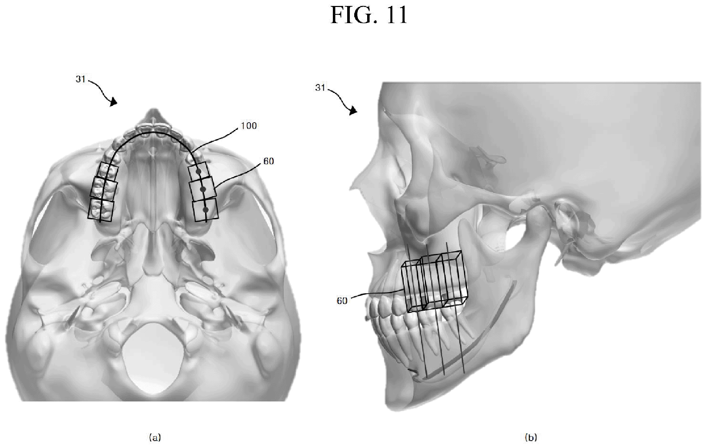

An embodiment of segmenting the anatomical structures will be described later with reference to FIG. 4. In addition, an embodiment of determining the size of the missing tooth area will be described later with reference to FIG. 6, and an embodiment of arranging the missing tooth area on an arch line will be described later with reference to FIG. 11.

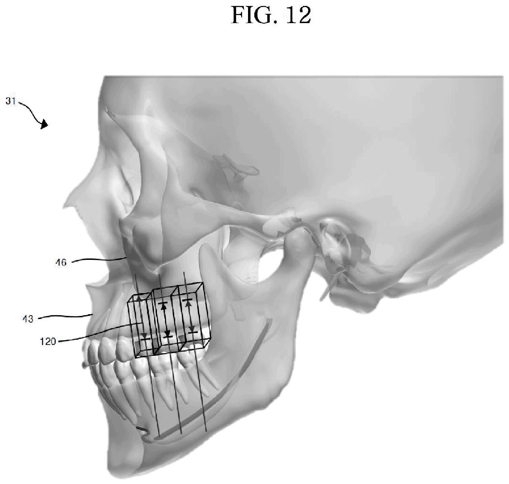

Subsequently, the apparatus 1 for designing a lateral approach surgical guide calculates a thickness of the residual bone on the basis of a distance between the maxillary sinus and the maxilla with respect to a central axis of the segmented missing tooth area (S230). An embodiment of calculating the thickness of the residual bone will be described later with reference to FIG. 12.

Subsequently, when the calculated thickness of the residual bone does not exceed a predetermined distance, the apparatus 1 for designing a lateral approach surgical guide forms a bone window on the basis of the segmented maxillary sinus (S240).

In step S240, when the calculated thickness of the residual bone does not exceed the predetermined distance, the apparatus 1 for designing a lateral approach surgical guide may recommend generation of a lateral approach surgical guide. Subsequently, upon receiving, from the user, a user operation signal selecting generation of the lateral approach surgical guide, the apparatus 1 for designing a lateral approach surgical guide may form a bone window on the basis of the segmented maxillary sinus.

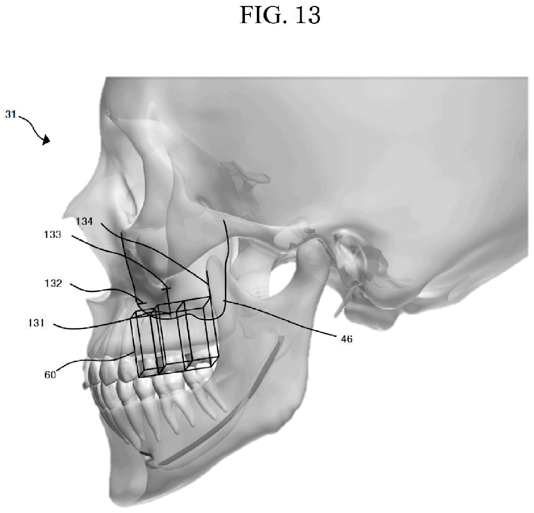

In step S240, the apparatus 1 for designing a lateral approach surgical guide may generate an inferior edge of the bone window at a position spaced superiorly by a first distance from an inferior border of the maxillary sinus, and generate an anterior edge of the bone window at a position spaced posteriorly by a second distance from an anterior border of the maxillary sinus. In addition, the apparatus 1 for designing a lateral approach surgical guide may generate a superior edge of the bone window at a position spaced superiorly by a third distance from the inferior edge of the bone window, and generate a posterior edge of the bone window at a position spaced posteriorly by a fourth distance from the anterior edge of the bone window. Subsequently, the apparatus 1 for designing a lateral approach surgical guide may connect reference points including the generated inferior edge, anterior edge, superior edge, and posterior edge of the bone window to form the bone window. An embodiment of forming the bone window will be described later with reference to FIGS. 13 to 15.

In step S240, when a septum is present in the dental imaging data, the apparatus 1 for designing a lateral approach surgical guide may form the bone window so as not to interfere with the septum.

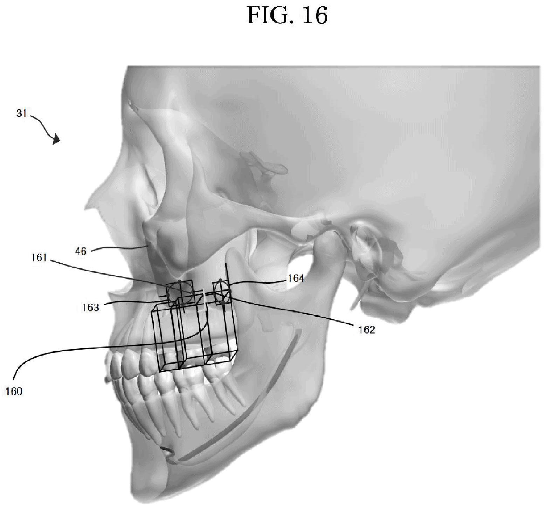

For example, the apparatus 1 for designing a lateral approach surgical guide may regard the septum as a posterior border of a bone and generate the posterior edge of the bone window at a predetermined distance anterior to the septum, and may regard the septum as an anterior border of a bone and generate the anterior edge of the bone window at a predetermined distance posterior to the septum. Subsequently, the apparatus 1 for designing a lateral approach surgical guide may additionally generate a superior edge and an inferior edge of the bone window, and connect reference points including the generated posterior edge, anterior edge, superior edge, and inferior edge of the bone window to form the bone window. An embodiment of forming the bone window in a case where a septum is present will be described later with reference to FIG. 16.

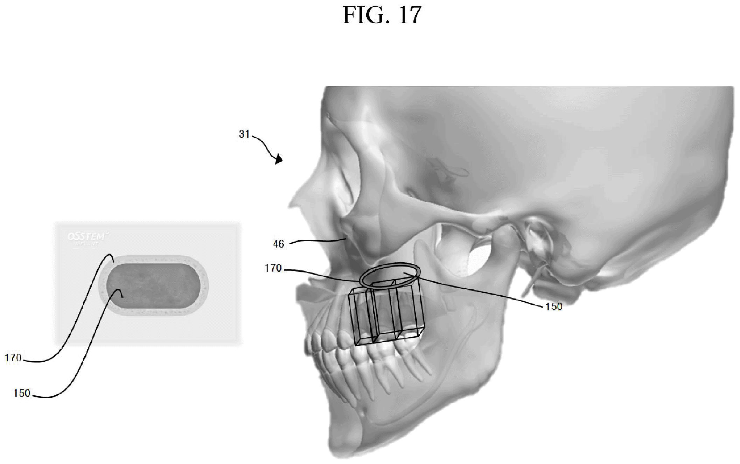

Subsequently, the apparatus 1 for designing a lateral approach surgical guide generates a lateral approach surgical guide including a guide rail formed at a predetermined distance from the periphery of the generated bone window to be larger than the bone window (S250). The reason for generating the guide rail is that a stopper of a cutting tool for forming the bone window needs to be received through the guide rail. Examples of the cutting tool include drills such as a core drill and a dome drill. The guide rail has a movement path in the form of a rail, and the cutting tool can remove bone of the oral cavity while coming into contact with the guide rail and moving along the movement path of the guide rail. A stopper of the cutting tool serves to prevent the cutting tool from advancing further when bone removal by the cutting tool has been performed up to a certain height. An embodiment of generating the guide rail will be described later with reference to FIG. 17.

In step S250, the apparatus 1 for designing a lateral approach surgical guide may determine the shape of the guide rail using the thickness of the residual bone and the length of the cutting tool.

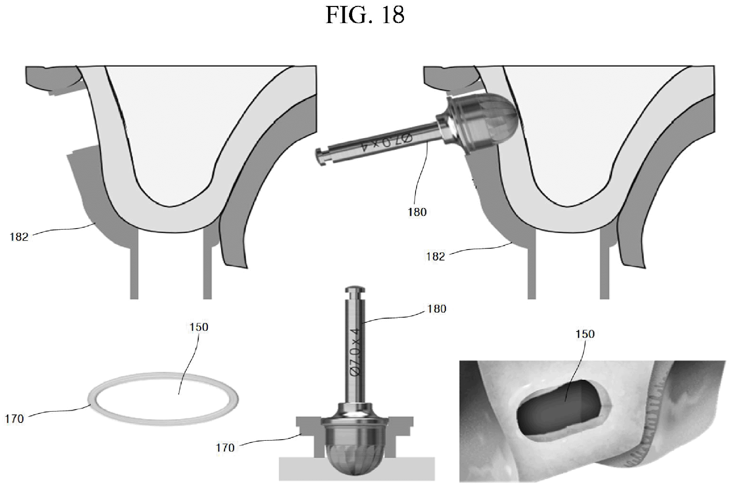

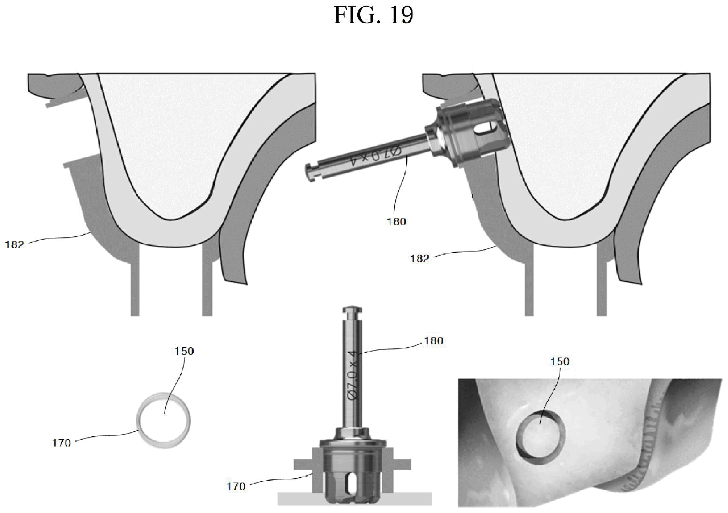

For example, when the thickness of the residual bone is greater than the length of the cutting tool, the apparatus 1 for designing a lateral approach surgical guide may generate the guide rail in a shape having a step on an inner side. In another example, when the thickness of the residual bone is less than the length of the cutting tool, the apparatus 1 for designing a lateral approach surgical guide may generate the guide rail in a shape protruding outward. An embodiment of determining the shape of the guide rail will be described later with reference to FIGS. 18 and 19.

In step S250, the apparatus 1 for designing a lateral approach surgical guide may generate the guide rail at a predetermined distance from the periphery of the bone window so as to be greater than the bone window such that a stopper of the cutting tool for lateral approach is allowed to be positioned thereon.

In step S250, the apparatus 1 for designing a lateral approach surgical guide may determine a thickness of the guide rail for forming the bone window in the lateral approach surgical guide by adjusting the step of the guide rail so that the stopper of the cutting tool can contact the guide rail at predefined discrete steps in accordance with the length of the stopper of the cutting tool and the thickness of the residual bone in the bone window formation area.

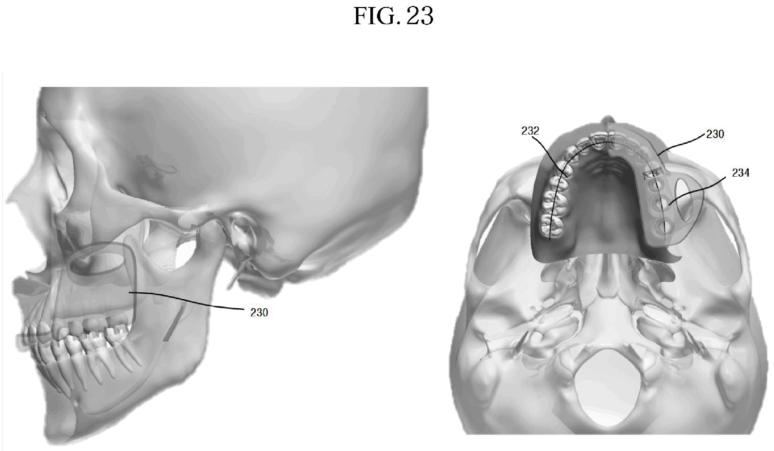

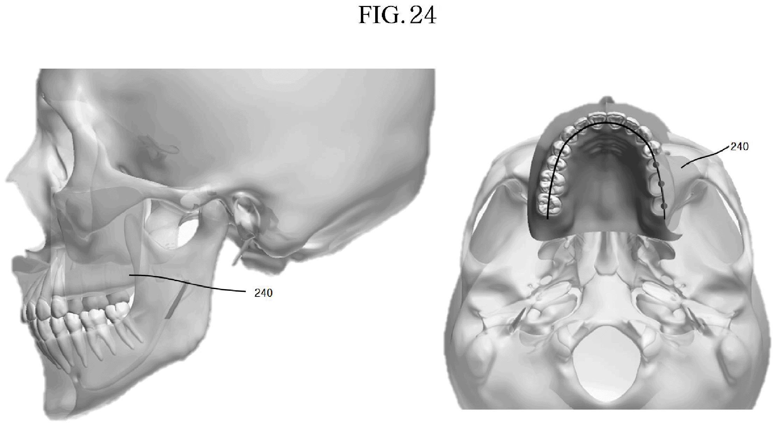

In step S250, the apparatus 1 for designing a lateral approach surgical guide may generate model data in which a maxillary sinus area, the missing tooth area, and a bone serving as a reference for an arch line are exposed. Subsequently, the apparatus 1 for designing a lateral approach surgical guide may generate the lateral approach surgical guide from the model data. At this time, the apparatus 1 for designing a lateral approach surgical guide may generate a bone-level lateral approach surgical guide for the bone window formation area. In contrast, the apparatus 1 for designing a lateral approach surgical guide may generate a gingival-level lateral approach surgical guide for a remaining area including a drilling hole area for placing an implant. An embodiment of generating the model data and generating the bone-level lateral approach surgical guide or the gingival-level lateral approach surgical guide will be described later with reference to FIGS. 23 and 24.

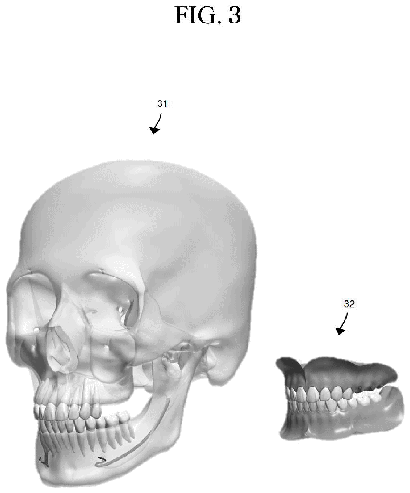

FIG. 3 illustrates CT data and scan data according to an embodiment of the present invention.

Referring to FIGS. 1 and 3, the apparatus 1 for designing a lateral approach surgical guide acquires dental imaging data of a patient. The apparatus 1 for designing a lateral approach surgical guide may acquire the dental imaging data by requesting the dental imaging data from an external device.

The dental imaging data may be CT data 31 and scan data 32. The scan data may be scan data acquired using an intraoral scanner. Alternatively, the scan data may be data obtained by scanning an impression after taking the impression using an impression material, or data obtained by pouring plaster into the impression to fabricate a plaster model and then scanning the plaster model.

FIG. 4 illustrates an anatomical structure segmentation screen according to an embodiment of the present invention.

Referring to FIGS. 1 and 4, the apparatus 1 for designing a lateral approach surgical guide may segment anatomical structures from dental imaging data, for example, first CT data 31. The apparatus 1 for designing a lateral approach surgical guide may segment the anatomical structures through an artificial neural network of artificial intelligence.

For example, the apparatus 1 for designing a lateral approach surgical guide acquires masking data for each anatomical structure of the patient from CT data of previous patients, and train an artificial neural network model by setting the acquired masking data as respective training data sets. Subsequently, the apparatus 1 for designing a lateral approach surgical guide acquires the patient's CT data, inputs the CT data into the artificial neural network model, and outputs an image in which the anatomical structures are segmented.

The anatomical structures may include a plurality of maxillary teeth 41, a plurality of mandibular teeth 42, a maxilla 43, a mandible 44, a nerve canal 45, a maxillary sinus 46, and the like. The maxilla 43 is used in the same sense as the maxilla bone. The maxillary sinus 46 refers to an air-filled space within the maxilla.

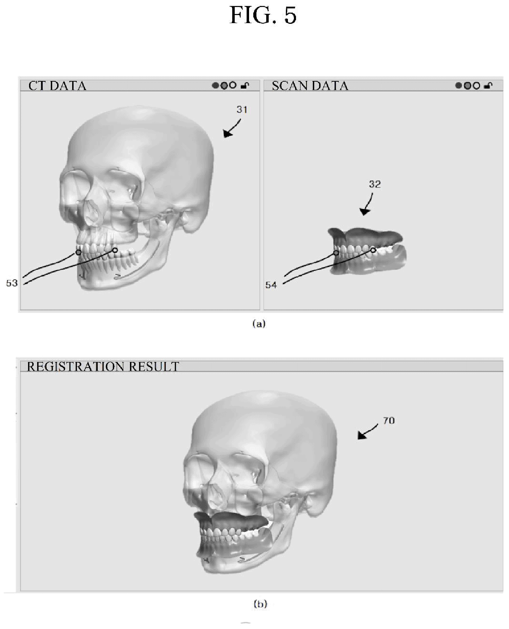

FIG. 5 illustrates a registration screen of CT data and scan data according to an embodiment of the present invention.

Referring to FIGS. 1 and 5, the apparatus 1 for designing a lateral approach surgical guide may register CT data 31 and scan data 32. The registration process between the CT data 31 and the scan data 32, which have different coordinate information, may vary depending on whether the patient is an edentulous case or a partially edentulous case.

For example, when the patient is edentulous, the apparatus 1 for designing a lateral approach surgical guide may perform registration by generating registration points on a resin marker.

In another example, when the patient is partially edentulous, the apparatus 1 for designing a lateral approach surgical guide may perform registration by receiving a user operation signal for selecting one point 53 and three points 54 of the teeth in the CT data 31 and the scan data 32, respectively, as shown in (a). The apparatus 1 for designing a lateral approach surgical guide generates and displays registration data 70 obtained by registering the CT data 31 and the scan data 32, as shown in (b).

FIG. 6 illustrates the size of a missing tooth area according to an embodiment of the present invention.

Referring to FIG. 6, when an implant placement site corresponds to a tooth in the posterior maxilla, the apparatus 1 for designing a lateral approach surgical guide analyzes the segmented maxillary sinus 46 and tooth regions 41 and 42, and calculates the thickness of the residual bone on that basis. The tooth region includes a missing tooth area 60.

First, the apparatus 1 for designing a lateral approach surgical guide determines the size of the missing tooth area 60 on the CT data 31 of the registration data.

When there is no tooth at a most posterior position, the apparatus 1 for designing a lateral approach surgical guide may determine the size of the missing tooth area 60 (bounding box) using an average tooth size. The size of the missing tooth area 60 includes buccal, lingual, mesial, and distal dimensions.

In another example, when a contralateral tooth exists on the opposite side of the missing tooth area 60, the apparatus 1 for designing a lateral approach surgical guide may determine the size of the missing tooth area 60 to be the same as the size of the contralateral tooth. An example of a case in which a contralateral tooth is present on the opposite side is when the maxillary right first molar and maxillary right second molar are missing while the maxillary left first molar and maxillary left second molar are present.

In another example, when teeth exist on both sides, the apparatus 1 for designing a lateral approach surgical guide may determine a vacant region between adjacent teeth as the size of the missing tooth area 60.

In another example, when there are a plurality of missing teeth, the apparatus 1 for designing a lateral approach surgical guide may determine the size of the missing tooth area 60 by substituting a space between adjacent teeth into an average tooth size.

Once the size of the missing tooth area 60 is determined, the apparatus 1 for designing a lateral approach surgical guide may generate the missing tooth area 60 as a hexahedron in 3D and as a rectangle in 2D, as shown in FIG. 6.

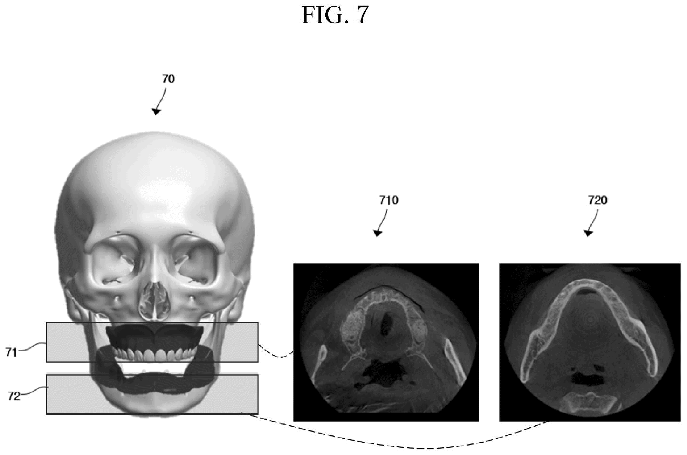

FIGS. 7 to 10 are views illustrating an arch line generation screen according to an embodiment of the present invention.

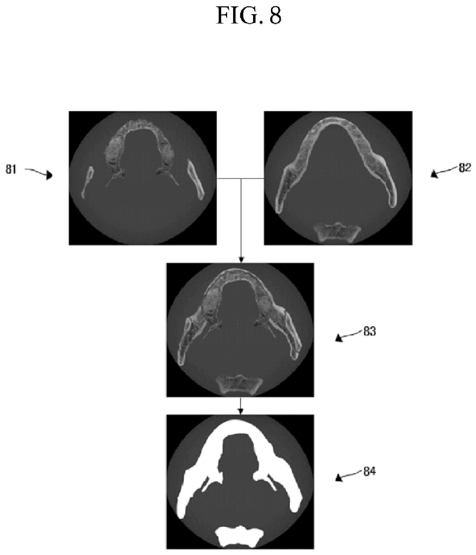

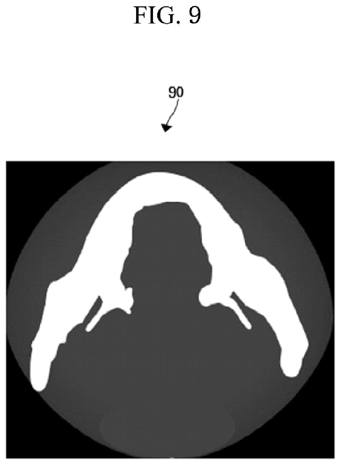

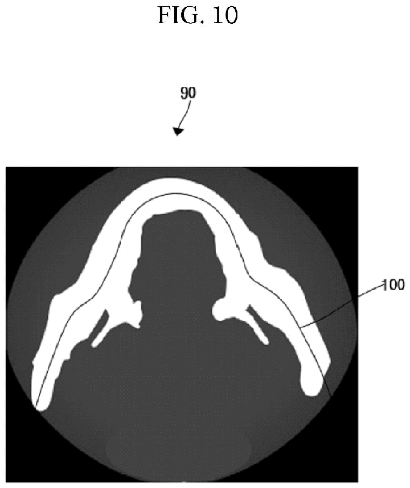

More specifically, FIG. 7 is a view illustrating a screen for separating the maxilla and mandible and extracting their respective images. FIG. 8 is a view illustrating a screen for generating a maximum intensity projection image. FIG. 9 is a view illustrating a screen for extracting the largest region among hard tissues from the maximum intensity projection image. FIG. 10 is a view illustrating a screen for generating an arch line in the maximum intensity projection image from which the largest region among hard tissues has been extracted.

Referring to FIGS. 1 and 7 to 10, the apparatus 1 for designing a lateral approach surgical guide generates an arch line after determining the size of the missing tooth area.

The apparatus 1 for designing a lateral approach surgical guide may generate the arch line using registration data 70. At this time, when the patient is partially edentulous, the apparatus 1 for designing a lateral approach surgical guide may use tooth information of the corresponding teeth. For example, the apparatus 1 for designing a lateral approach surgical guide segments the teeth and forms a hexahedron in 3D and a rectangle in 2D on the segmented tooth. Subsequently, the apparatus 1 for designing a lateral approach surgical guide determines, as a tooth axis, a point passing through a predetermined point (e.g., the exact center) in the hexahedron in 3D or the rectangle in 2D. Subsequently, the apparatus 1 for designing a lateral approach surgical guide uses the determined tooth axis to generate the arch line. In contrast, when the patient is edentulous, the apparatus 1 for designing a lateral approach surgical guide may generate a maximum intensity projection image in an axial view in order to generate a point for generating the arch line in the missing tooth area. The missing tooth area may be, for example, teeth #25 (maxillary left second premolar), #26 (maxillary left first molar), and #27 (maxillary left second molar).

Hereinafter, the process of generating the arch line will be described in more detail.

As shown in FIG. 7, the apparatus 1 for designing a lateral approach surgical guide may extract a maxillary image 710 and a mandibular image 720 by segmenting a maxillary plane 71 and a mandibular plane 72 from the registration data 70.

Subsequently, as shown in FIG. 8, the apparatus 1 for designing a lateral approach surgical guide extracts hard tissue from the maxillary image 710 and the mandibular image 720 of FIG. 7 and generate hard tissue images 81 and 82. For example, the apparatus 1 for designing a lateral approach surgical guide extracts hard tissues having a brightness value set to N (e.g., 255) from the maxillary image 710 and the mandibular image 720. The hard tissues may include teeth, bone, implants, and the like.

Subsequently, the apparatus 1 for designing a lateral approach surgical guide generates a combined image 83 by accumulating the maxillary image and mandibular image that are composed of the extracted hard tissues.

Subsequently, the apparatus 1 for designing a lateral approach surgical guide generates a maximum intensity projection image 84, which is a 2D representation of the accumulated image 83. The maximum intensity projection image 84 is expressed as a 2D image in which cross-sections from the bone level of each of the maxilla and mandible to the lowest bone level, with respect to the occlusal plane, are combined. Accordingly, 3D data can be expressed in 2D through the maximum intensity projection image 84.

Subsequently, as shown in FIG. 9, the apparatus 1 for designing a lateral approach surgical guide generates an image 90 by extracting the largest region among the hard tissues from the maximum intensity projection image 84. Through this, the tooth portion and jawbone among the hard tissues remain, while the cervical spine portion is removed.

Subsequently, as shown in FIG. 10, the apparatus 1 for designing a lateral approach surgical guide generates an arch line 100 in the maximum intensity projection image 90 from which the largest region among the hard tissues. For example, the apparatus 1 for designing a lateral approach surgical guide may generate the arch line 100 at the exact center based on one-half of the bone width in the maximum intensity projection image 90. Once the arch line 100 is generated, the shape of the arch line 100 can be viewed on the previously set occlusal plane. When the maximum intensity projection image is generated in the axial direction, the the maxilla and mandible appear together as if in a single plane, so that the images of the maxilla and mandible overlap, resulting in a single arch line 100 being generated.

FIG. 11 illustrates a screen for arranging a missing tooth area on an arch line according to an embodiment of the present invention.

Referring to FIGS. 1 and 11, the apparatus 1 for designing a lateral approach surgical guide arranges the missing tooth area 60 on the arch line 100 on the CT data 31 of the registration data 70. The apparatus 1 for designing a lateral approach surgical guide may arrange the missing tooth area 60 on the arch line 100 by using, as a central axis, a point passing through the exact center of the hexahedron in 3D that corresponds to the missing tooth area 60.

FIG. 12 illustrates a screen for calculating a thickness of a residual bone according to an embodiment of the present invention.

Referring to FIGS. 1 and 12, the apparatus 1 for designing a lateral approach surgical guide calculates a thickness of the residual bone 120 as a distance, on the central axis of the missing tooth area 60, between the maxillary sinus 46 and the maxilla 43 segmented in the CT image.

When there is at least one missing tooth area where the calculated thickness of the residual bone 120 does not exceed a predetermined distance, for example, within 4 mm, the apparatus 1 for designing a lateral approach surgical guide may recommend generation of a lateral approach surgical guide.

FIGS. 13 to 15 illustrate a screen for generating a bone window according to an embodiment of the present invention.

Referring to FIGS. 1 and 13, when the calculated thickness of the residual bone 120 does not exceed a predetermined distance, for example, 4 mm, the apparatus 1 for designing a lateral approach surgical guide recommends generation of a lateral approach surgical guide.

Subsequently, upon receiving, from the user, a user operation signal selecting generation of the lateral approach surgical guide, the apparatus 1 for designing a lateral approach surgical guide forms a bone window on the basis of the maxillary sinus 46 segmented from the CT image.

For example, the apparatus 1 for designing a lateral approach surgical guide generates an inferior edge 131 of the bone window at a position spaced superiorly by a first distance, for example, 2 mm to 3 mm, from an inferior border, that is, the lowest end, of the segmented maxillary sinus 46.

Subsequently, the apparatus 1 for designing a lateral approach surgical guide generates an anterior edge 132 of the bone window at a position spaced posteriorly by a second distance, for example, 2 mm to 3 mm, from an anterior border, that is, a most anterior end, of the segmented maxillary sinus 46.

Subsequently, the apparatus 1 for designing a lateral approach surgical guide generates a superior edge 133 of the bone window at a position spaced superiorly by a third distance, for example, 5 mm, from the inferior edge 131 of the bone window. Here, the third distance may correspond to a diameter of the cutting tool.

Subsequently, the apparatus 1 for designing a lateral approach surgical guide generates a posterior edge 134 of the bone window at a position spaced posteriorly by a fourth distance, for example, 10 mm to 15 mm, from the anterior edge 132 of the bone window.

When a tooth is present at a most posterior position, the apparatus 1 for designing a lateral approach surgical guide may generate the posterior edge 134 of the bone window on the basis of the front side of a most posterior tooth region, which is represented as a hexahedron in 3D and as a rectangle in 2D. For example, the apparatus 1 for designing a lateral approach surgical guide may generate the posterior edge 134 of the bone window at a predetermined distance, for example, 2 mm to 3 mm anterior to the most posterior tooth.

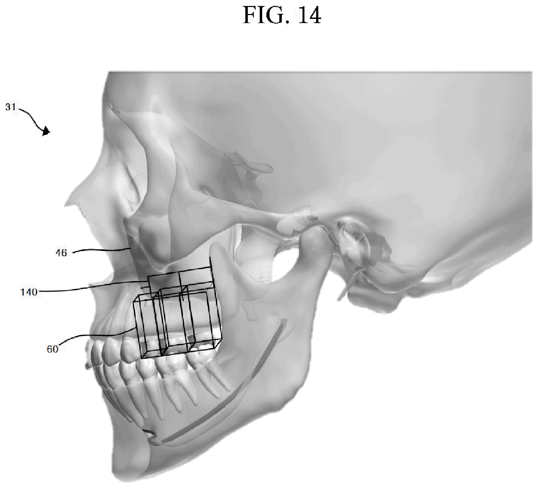

After generating reference points including the inferior edge 131, anterior edge 132, superior edge 133, and posterior edge 134 of the bone window, the apparatus 1 for designing a lateral approach surgical guide connects the reference points 131, 132, 133, and 134 with lines to generate a quadrilateral 140, as shown in FIG. 14.

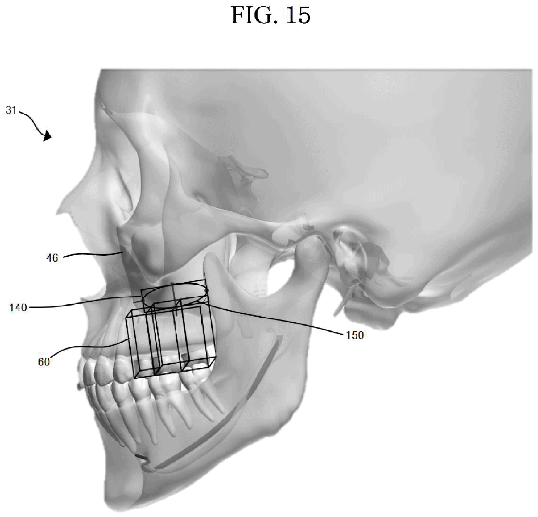

Subsequently, as shown in FIG. 15, the apparatus 1 for designing a lateral approach surgical guide generates, within the quadrilateral 140, an ellipse 150 that passes through each of the reference points 131, 132, 133, and 134. The generated ellipse 150 finally becomes the bone window.

FIG. 16 illustrates a screen for generating a bone window in a case where a septum is present according to an embodiment of the present invention.

Referring to FIGS. 1 and 16, when a septum 160 is present in the data, the apparatus 1 for designing a lateral approach surgical guide may generate a bone window in a different manner. Since the septum 160 is protruding bone, the bone window in the form of an ellipse 150 is generated so as to avoid the septum 160.

For example, when the septum 160 is present, the apparatus 1 for designing a lateral approach surgical guide may regard the septum 160 as a posterior border of a bone and, in the same manner as for the anterior edge, generate a posterior edge 161 of the bone window at a predetermined distance, for example, 2 mm to 3 mm, anterior to the septum 160.

Subsequently, the apparatus 1 for designing a lateral approach surgical guide may regard the septum 160 as an anterior border of a bone and, in the same manner as for the posterior edge, generate an anterior edge 162 of the bone window at a predetermined distance, for example, 2 mm to 3 mm posterior to the septum 160.

Subsequently, the apparatus 1 for designing a lateral approach surgical guide additionally generates a superior edge and an inferior edge of the bone window.

Thereafter, the apparatus 1 for designing a lateral approach surgical guide generates a final bone window by connecting reference points including the posterior edge, anterior edge, superior edge, and inferior edge of the bone window to form a quadrilateral, and generating, within the quadrilateral, an ellipse passing through each reference point.

The method of determining the superior edge and inferior edge of the bone window and the method of generating the bone window by connecting the reference points are as described above with reference to FIGS. 13 to 15.

For example, the apparatus 1 for designing a lateral approach surgical guide generates a first quadrilateral including the posterior edge 161 and a second quadrilateral including the anterior edge 162 so as to avoid the septum 160.

Subsequently, the apparatus 1 for designing a lateral approach surgical guide generates a first ellipse 163 passing through respective reference points within the first quadrilateral and a second ellipse 164 passing through respective reference points within the second quadrilateral. In this case, the first ellipse 163 and the second ellipse 164 ultimately become the bone windows.

FIG. 17 illustrates a screen for generating a bone window and a guide rail in a case where a septum is present according to an embodiment of the present invention.

Referring to FIGS. 1 and 17, the apparatus 1 for designing a lateral approach surgical guide generates a guide rail 170 which is formed at a predetermined distance, for example, 0.5 mm, from the periphery of the finally generated bone window 150 so as to be larger than the bone window 150. The reason for generating the guide rail 170 is that a stopper of a cutting tool for forming the bone window 150 is to be received through the guide rail 170 so that the bone window 150 can be formed as designed. Examples of the cutting tool include drills such as a core drill and a dome drill. The guide rail 170 has a movement path in the form of a rail, and the cutting tool can remove bone of the oral cavity while coming into contact with the guide rail 170 and moving along the movement path of the guide rail 170. The stopper of the cutting tool or the guide rail 170 serves to prevent the cutting tool 180 from advancing further when bone removal by the cutting tool 180 has been performed up to a certain height.

FIGS. 18 and 19 illustrate various shapes of a guide rail determined according to the length of a cutting tool according to various embodiments of the present invention.

More specifically, FIG. 18 is a view illustrating a configuration in which a step of the guide rail is formed inwardly, and FIG. 19 is a view illustrating a configuration in which the guide rail protrudes outward.

Referring to FIGS. 1, 18, and 19, the lateral approach surgical guide 182 includes a guide rail 170, and the stopper of the cutting tool 180 may remove bone of the oral cavity while coming into contact with the guide rail 170 and moving along the movement path of the guide rail 170.

The thickness of the bone window 150 corresponds to the diameter of the cutting tool 180, and the guide rail 170 is required to be formed larger than the bone window 150 by an additional predetermined distance, for example, 0.5 mm, so that the stopper of the cutting tool 180 can contact the guide rail 170 of the lateral approach surgical guide 182.

As shown in FIG. 18, the apparatus 1 for designing a lateral approach surgical guide forms a step on the guide rail 170 so that the stopper of the cutting tool 180 can contact the guide rail 170 of the lateral approach surgical guide 182. The thickness of the step portion of the guide rail 170 is adjusted according to the thickness of the residual bone. Accordingly, the apparatus 1 for designing a lateral approach surgical guide can uniformly generate the lateral approach surgical guide 182 with a predetermined thickness, for example, a thickness of 2 mm. The cutting tool 180 of FIG. 18 corresponds to a core drill.

Referring to FIGS. 1 and 19, the apparatus 1 for designing a lateral approach surgical guide uniformly generates the lateral approach surgical guide 182 with a predetermined thickness, for example, a thickness of 2 mm. When bone is to be removed using the cutting tool 180 in order to form a bone window, if the thickness of the residual bone is less than the length of the cutting tool 180, the apparatus 1 for designing a lateral approach surgical guide generates the guide rail 170 in a shape protruding outward. Accordingly, there is an advantage that the cutting tool 180 can form the bone window 150 in a manner exactly matching the outwardly protruding guide rail 170. The cutting tool 180 of FIG. 19 corresponds to a dome drill.

The apparatus 1 for designing a lateral approach surgical guide determines the shape of the guide rail 170 for forming the bone window 150 according to the length of the cutting tool 180. A height of the lateral approach surgical guide 182 is a height obtained by subtracting the thickness of the residual bone from the length of the cutting tool 180. When the thickness of the residual bone is greater than the length of the cutting tool 180, the apparatus 1 for designing a lateral approach surgical guide generates the guide rail 170 in a shape having a step on an inner side. In contrast, when the thickness of the residual bone is less than the length of the cutting tool 180, the apparatus 1 for designing a lateral approach surgical guide generates the guide rail 170 in a shape protruding outward.

For example, as shown in FIG. 18, when the length of the cutting tool 180 is 4 mm and the thickness of the residual bone for forming an actual bone window is 5 mm, the apparatus 1 for designing a lateral approach surgical guide generates the guide rail 170 in a shape in which a step is formed inwardly.

In contrast, as shown in FIG. 19, when the length of the cutting tool 180 is 4 mm and the thickness of the residual bone for forming the bone window 150 is 2 mm, the apparatus 1 for designing a lateral approach surgical guide generates the guide rail 170 in a shape protruding outward.

Also, there are cases, such as when using a core drill, in which the bone window 150 needs to be widened by advancing the cutting tool 180 along the guide rail 170 while the cutting tool 180 is brought into proximity with the guide rail 170. Such a case occurs when the bone has a non-uniform thickness. In this case, the apparatus 1 for designing a lateral approach surgical guide may generate the guide rail 170 for forming the bone window 150 in a structure having steps in predetermined increments, for example, in increments of 1 mm.

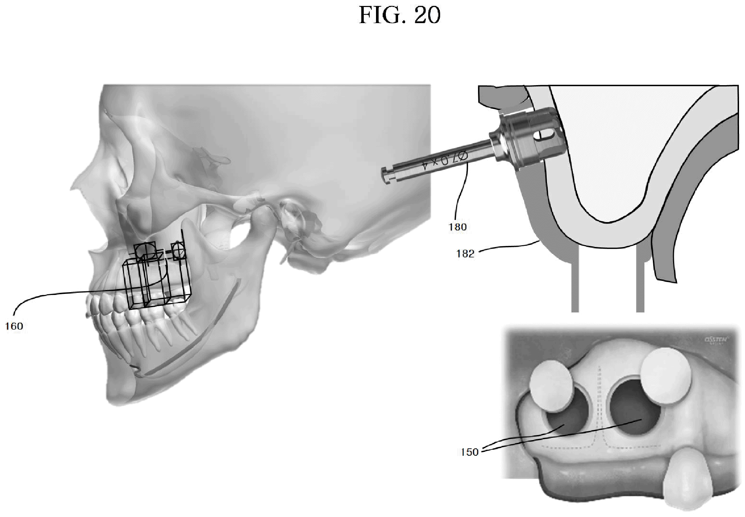

FIG. 20 illustrates a screen for generating a guide rail in a case where a septum is present according to an embodiment of the present invention.

Referring to FIGS. 1 and 20, when a septum 160 is present, the apparatus 1 for designing a lateral approach surgical guide loads a cutting tool 180 having a predetermined length from a library so that the bone window 150 can be formed while avoiding the septum 160. In addition, the apparatus 1 for designing a lateral approach surgical guide generates the guide rail by using the length of the cutting tool 180 and the thickness of residual bone.

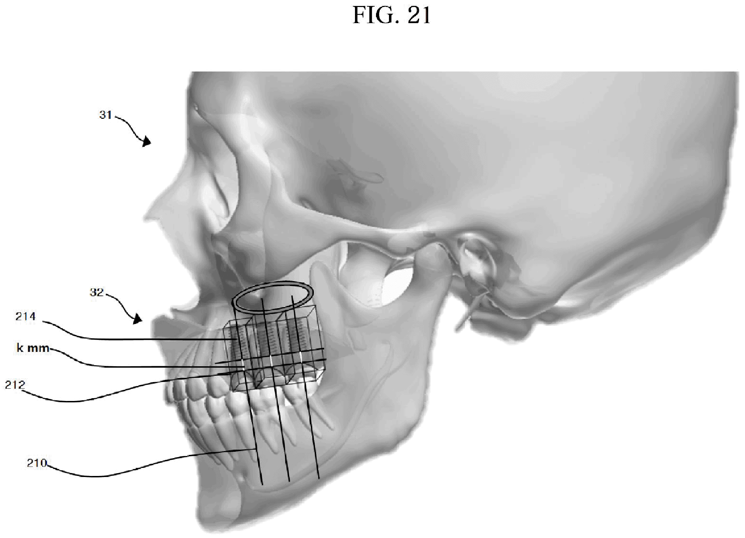

FIG. 21 illustrates an implant placement screen according to an embodiment of the present invention.

Referring to FIGS. 1 and 21, the apparatus 1 for designing a lateral approach surgical guide may place an implant 214 on the basis of a central axis 210 of the missing tooth area. In this case, the apparatus 1 for designing a lateral approach surgical guide may place the implant 214 so that the uppermost end of the implant 214 is located at a position spaced inferiorly, by a predetermined distance, for example, k=3 mm, from a point at which the central axis 210 of the missing tooth area meets gingiva 212 of the scan data 32 of the registration data.

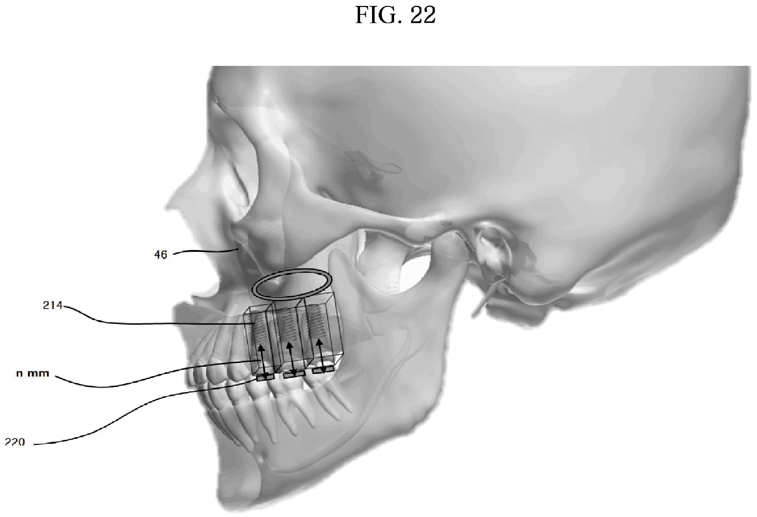

FIG. 22 illustrates a drilling hole formation screen according to an embodiment of the present invention.

Referring to FIGS. 1 and 22, the apparatus 1 for designing a lateral approach surgical guide may form a drilling hole 220 for implant placement at a position spaced superiorly, by a predetermined distance, for example, n=10.5 mm, from the placed implant 214. The apparatus 1 for designing a lateral approach surgical guide may form the drilling hole 220 by user selection only in a case where the implant 214 is to be placed immediately after performing the maxillary sinus lift procedure using the lateral approach.

FIG. 23 illustrates a screen for generating a gingival-level lateral approach surgical guide according to an embodiment of the present invention, and FIG. 24 illustrates a screen for generating a bone-level lateral approach surgical guide according to an embodiment of the present invention.

Referring to FIGS. 1, 23, and 24, the apparatus 1 for designing a lateral approach surgical guide generates a lateral approach surgical guide on the basis of the maxilla in order to perform a maxillary sinus lift procedure using the lateral approach. During surgery, an operator incises and reflects the gingiva, forms the bone window along the guide rail of the lateral approach surgical guide, places the implant, and then sutures the gingiva. Accordingly, the apparatus 1 for designing a lateral approach surgical guide determines an area for forming the bone window so as to conform to the maxilla by performing a lateral approach on the basis of the arch line (that is, a bone window formation area).

The apparatus 1 for designing a lateral approach surgical guide may generate a bone-level lateral approach surgical guide 240 for the bone window formation area. The "bone level" means generating the lateral approach surgical guide 240 in conformity with the bone.

In addition, the apparatus 1 for designing a lateral approach surgical guide may generate a gingival-level lateral approach surgical guide 230 for the remaining area including the drilling hole area 234 on the basis of an arch line 232, except for a bone window formation area. The "gingival-level" means generating the lateral approach surgical guide 230 in conformity with the gingiva.

The apparatus 1 for designing a lateral approach surgical guide may generate new model data in which a maxillary sinus area, the missing tooth area, and a bone serving as a reference for the arch line are exposed. In general, an incision line is made along the arch line, and, since remaining portions of a guide template other than the bone window formation area are in contact with the gingiva and teeth, the apparatus 1 for designing a lateral approach surgical guide generates the gingival-level lateral approach surgical guide 230.

After generating the model data, the apparatus 1 for designing a lateral approach surgical guide may generate the bone-level lateral approach surgical guide 240 for the bone window formation area and the gingival-level lateral approach surgical guide 230 for the remaining area.

The apparatus 1 for designing a lateral approach surgical guide may determine the thickness of the guide rail for forming the bone window in the bone-level lateral approach surgical guide 240 by adjusting the step of the guide rail such that the stopper of the cutting tool can contact the guide rail at predefined discrete steps in accordance with the length of the cutting tool and the thickness of the residual bone in the bone window formation area.

The apparatus 1 for designing a lateral approach surgical guide may generate the bone-level lateral approach surgical guide 240 such that the bone-level lateral approach surgical guide 240 has a thickness greater than or equal to a predetermined length, for example, a thickness of 2 mm. The "bone-level" means generating the bone-level lateral approach surgical guide 240 in conformity with the bone. Since the stopper of the cutting tool comes into contact with the guide rail of the lateral approach surgical guide at discrete steps of 1 mm, the apparatus 1 for designing a lateral approach surgical guide also sets the thickness of the bone-level lateral approach surgical guide 240 to correspond to the discrete steps.

So far, the present invention has been described with reference to embodiments thereof. It will be understood by those skilled in the art that various changes in form and details may be made therein without departing from the essential features of the present invention. Therefore, the disclosed embodiments should be considered in descriptive sense only and not for purposes of limitation. The scope of the present invention should be defined not by the detailed description but by the appended claims, and all differences falling within a scope equivalent to the claims should be construed as being encompassed by the present invention.

## Claims

1. A method for designing a lateral approach surgical guide using an apparatus for designing a lateral approach surgical guide, the method comprising:
acquiring dental imaging data of a patient;
segmenting, from the acquired dental imaging data, a tooth region including a missing tooth area, and anatomical structures including a maxillary sinus and a maxilla;
calculating a thickness of a residual bone on the basis of a distance, measured with respect to a central axis of the missing tooth area, between the maxillary sinus and the maxilla among the segmented anatomical structures;
forming a bone window on the basis of the segmented maxillary sinus when the calculated thickness of the residual bone does not exceed a predetermined distance; and
generating a lateral approach surgical guide including a guide rail formed at a predetermined distance from a periphery of the formed bone window so as to be larger than the bone window.

2. The method of claim 1, further comprising:
receiving an implant placement site and a treatment plan; and
providing a lateral approach surgical mode when the implant placement site is in a posterior maxilla and the treatment plan is for a maxillary sinus lift procedure.

3. The method of claim 1, wherein the segmenting of the anatomical structures comprises:
determining a size of the missing tooth area in the dental imaging data;
displaying the missing tooth area having the determined size as a hexahedron in 3D and as a rectangle in 2D; and
arranging the missing tooth area on an arch line.

4. The method of claim 3, wherein the determining of the size of the missing tooth area comprises, when there is no tooth at a most posterior position, determining the size of the missing tooth area using an average tooth size from the dental imaging data, and when a contralateral tooth exists on an opposite side of the missing tooth area, determining the size of the missing tooth area to be the same as a size of the contralateral tooth.

5. The method of claim 3, wherein the determining of the size of the missing tooth area comprises, where teeth are present on both sides of the missing tooth area, determining a vacant region between adjacent teeth as the size of the missing tooth area.

6. The method of claim 3, wherein the determining of the size of the missing tooth area comprises, when there are a plurality of missing teeth, determining the size of the missing tooth area by substituting a space between adjacent teeth into an average tooth size.

7. The method of claim 1, wherein the forming of the bone window comprises:
generating an inferior edge of the bone window at a position spaced superiorly by a first distance from an inferior border of the maxillary sinus;
generating an anterior edge of the bone window at a position spaced posteriorly by a second distance from an anterior border of the maxillary sinus;
generating a superior edge of the bone window at a position spaced superiorly by a third distance from the inferior edge of the bone window;
generating a posterior edge of the bone window at a position spaced posteriorly by a fourth distance from the anterior edge of the bone window; and
connecting reference points including the generated inferior edge, anterior edge, superior edge, and posterior edge of the bone window to form a quadrilateral, and generating, within the quadrilateral, an ellipse passing through each of the reference points to form the bone window.

8. The method of claim 7, wherein the forming of the bone window comprises, when a tooth is present at a most posterior position, generating the posterior edge of the bone window at a predetermined distance anterior to a region of the most posterior tooth.

9. The method of claim 1, wherein the forming of the bone window comprises:
when a septum is present in the dental imaging data, generating a posterior edge of the bone window at a predetermined distance anterior to the septum while regarding the septum as a posterior border of a bone;
generating an anterior edge of the bone window at a predetermined distance posterior to the septum while regarding the septum as an anterior border of a bone;
additionally generating a superior edge and an inferior edge of the bone window; and
connecting reference points including the generated posterior edge, anterior edge, superior edge, and inferior edge of the bone window to form the bone window.

10. The method of claim 1, wherein the generating of the lateral approach surgical guide comprises determining a shape of the guide rail using the thickness of the residual bone and a length of a cutting tool.

11. The method of claim 10, wherein the generating of the lateral approach surgical guide comprises:
when the thickness of the residual bone is greater than the length of the cutting tool, generating the guide rail in a shape having a step on an inner side; and
when the thickness of the residual bone is less than the length of the cutting tool, generating the guide rail in a shape protruding outward.

12. The method of claim 1, wherein the generating of the lateral approach surgical guide comprises generating the guide rail at the predetermined distance from the periphery of the bone window so as to be greater than the bone window such that a stopper of a cutting tool for lateral approach is allowed to be positioned thereon.

13. The method of claim 1, wherein the generating of the lateral approach surgical guide comprises:
generating model data in which a maxillary sinus area, the missing tooth area, and a bone serving as a reference for an arch line are exposed; and
generating the lateral approach surgical guide from the model data.

14. The method of claim 13, wherein the generating of the lateral approach surgical guide from the model data comprises generating a bone-level lateral approach surgical guide for a bone window formation area, and generating a gingival-level lateral approach surgical guide for a remaining area including a drilling hole area for placing an implant.

15. The method of claim 1, wherein the generating of the lateral approach surgical guide comprises determining a thickness of the guide rail for forming the bone window in the lateral approach surgical guide by adjusting a step of the guide rail such that a stopper of a cutting tool can contact the guide rail at predefined discrete steps in accordance with a length of the stopper of the cutting tool and the thickness of the residual bone in a bone window formation area.

16. The method of claim 1, further comprising:
recommending generation of the lateral approach surgical guide when the calculated thickness of the residual bone does not exceed a predetermined distance; and
forming the bone window on the basis of the segmented maxillary sinus upon receiving, from a user, a user operation signal selecting generation of the lateral approach surgical guide.

17. An apparatus for designing a lateral approach surgical guide comprising:
a data acquisition unit configured to acquire dental imaging data for lateral approach surgery;
a control unit configured to segment, from the acquired dental imaging data, a tooth region including a missing tooth area and anatomical structures including a maxillary sinus and a maxilla, calculate a thickness of a residual bone on the basis of a distance between the maxillary sinus and the maxilla with respect to a central axis of the missing tooth area among the segmented anatomical structures, form a bone window on the basis of the segmented maxillary sinus when the calculated thickness of the residual bone does not exceed a predetermined distance, and generate a lateral approach surgical guide including a guide rail formed at a predetermined distance from a periphery of the generated bone window so as to be larger than the bone window; and
a display unit configured to display data including the lateral approach surgical guide.
